# EUROPEAN PATENT APPLICATION

(11) **EP 3 214 598 A1**
(43) Date of publication of application: **06.09.2017**
(21) Application number: 15855044.2
(22) Date of filing: 07.10.2015
(51) Int. Cl.: G06Q 50/22

(54) **MEDICAL INFORMATION MANAGEMENT SERVER DEVICE AND METHOD FOR MANAGING MEDICAL INFORMATION**

(30) Priority: 31.10.2014 JP 2014222856
(71) Applicant: Kimura, Masashi, Nishinomiya-shi, Hyogo 669-1103 (JP)
(72) Inventor: Kimura, Masashi, Nishinomiya-shi, Hyogo 669-1103 (JP)
(74) Representative: Viering, Jentschura & Partner mbB Patent- und Rechtsanwälte
(86) International application number: PCT/JP2015/078505
(87) International publication number: WO 2016/067862

(57) **Abstract**

A medical information management server (1) is connected to terminal devices (2) in a communicable manner, manages examination result information for medical use, and incudes a product number receiving section (101), a key generating section (102), a storage region setting section (103), and a key transmitting section (104). The product number receiving section (101) receives product number information of a medical product from one (21) of the terminal devices through the network (3). When the product number receiving section (101) receives the product number information, the key generating section (104) generates authentication key information based on the product number information. The storage region setting section (103) sets a storage region for storing examination result information in correspondence with the authentication key information generated by the key generating section (104). The key transmitting section (104) transmits the authentication key information to the terminal device (21) through the network (3).

## Description

### [TECHNICAL FIELD]

The present invention relates to medical information management servers and medical information management methods for managing information on medical, care, and welfare services containing examination result information for medical use.

### [BACKGROUND ART]

A medical information management server has been known traditionally. The medical information management server is connected to a plurality of terminal devices through a network in a communicable manner and manages examination result information for medical use.

A server used for examination and diagnosis that a plurality of health care workers carry out in liaison with one another has been disclosed among such medical information management servers (see Patent Literature 1). The server disclosed in Patent Literature 1 receives basic data, MRI brain image data, and electrocardiogram data of a patient from an attending physician's terminal device. Upon receiving the MRI brain image data, the server transmits image diagnosis instruction data to a radiologist's terminal device and receives image diagnosis result data from the radiologist's terminal device in response. The server further receives blood test result data from a terminal device of a blood test center. Once the server receives all of the basic data, the MRI brain image data, the electrocardiogram data, the blood test result data, and the image diagnosis result data, the server generates comprehensive diagnosis instruction data and transmits it to a neurologist's terminal device.

Patent Literature 1 discloses that data transmission and receipt are performed in a unitary manner through the server in a situation in which the plurality of health care workers work in liaison with one another, resulting in reduction in occurrence of mistakes such as confusion between examinees.

### [CITATION LIST]

### [Patent Literature]

[Patent Literature] Japanese Patent Application Laid-Open Publication No.2011-164956

### [SUMMARY OF INVENTION]

### [Technical Problem]

However, in the server disclosed in Patent Literature 1, clicking for example a "comprehensive diagnosis start" button displayed on a display section of the neurologist's terminal device can enable acquisition of for example the electrocardiogram data and the image diagnosis result data from the server. This may involve leakage of various examination result information for medical use.

The present invention has been made in view of the foregoing problem and has its object of providing a medical information management server and a medical information management method that can prevent leakage of information on medical, care, and welfare services containing examination result information.

### [Solution to Problem]

A first medical information management server according to the present invention is connected to a plurality of terminal devices in a communicable manner through a network, manages information on medical, care, and welfare services containing examination result information for medical use, and includes a product number receiving section, a key generating section, a storage region setting section, and a key transmitting section. The product number receiving section receives product number information of a medical product from a first terminal device among the plurality of terminal devices through the network. The key generating section generates authentication key information based on the product number information when the product number receiving section receives the product number information. The storage region setting section sets a storage region for storing examination result information in correspondence with the authentication key information generated by the key generating section. The key transmitting section transmits the authentication key information to the first terminal device through the network.

A second medical information management according to the present invention is the first medical information management server, wherein the medical product to which a code is attached, and the code indicates information containing the product number information and email address information of the medical information management server. Further, the product number receiving section receives the product number information in a manner to receive an email containing the product number information from the first terminal device.

A third medical information management server according to the present invention is the second medical information management server, wherein the code is a barcode attached to a package of the medical product.

A fourth medical information management server according to the present invention is the second or third medical information management server, wherein the medical product is used for an examination, and once the medical product is used in the examination for a single patient, the medical product is not used in the examination for another patient.

A fifth medical information management server according to the present invention is any one of the first to fourth medical information management servers that further includes an examination result receiving section and an examination result recording section. The examination result receiving section receives authentication key information, patient identification information, physician identification information, and examination result information from a second terminal device among the plurality of terminal devices through the network, wherein the patient identification information is information used for identifying a patient subjected to an examination and the physician identification information is information used for identifying a physician who has instructed the examination. When the examination result receiving section receives the authentication key information, the patient identification information, the physician identification information, and the examination result information, the examination result recording section stores the received examination result information in a storage region corresponding to the received authentication key information in association with the received authentication key information, the received patient identification information, and the received physician identification information.

A sixth medical information management server according to the present invention is the fifth medical information management server, wherein the examination result recording section performs the following processing when the examination result receiving section receives authentication key information, patient identification information, physician identification information, and examination result information. That is, in a situation in which when the examination result receiving section receives authentication key information, patient identification information, physician identification information, and examination result information, no storage region corresponding to the received authentication key information is set, the examination result recording section transmits a message indicating that the received authentication key information is wrong to the second terminal device.

A seventh medical information management server according to the present invention is the fifth or sixth medical information management server that includes a provision request receiving section, a first determination section, and an information providing section. The provision request receiving section receives authentication key information, patient identification information, and information indicating a request for provision of examination result information from a third terminal device among the plurality of terminal devices through the network. The first determination section, when the provision request receiving section receives the authentication key information, the patient identification information, and the information indicating a request for provision of examination result information, performs determination as to whether or not the received patient identification information matches the patient identification information associated with the received authentication key information. The information providing section transmits examination result information stored in a storage region corresponding to the authentication key information received by the provision request receiving section to the third terminal device upon the first determination section determining match.

An eighth medical information management server according to the present invention is the seventh medical information management server, wherein the examination result information contains auxiliary information including at least one piece of medical institution information indicating a medical institution in which an examination has been carried out, contact address information indicating a contact address of the medical institution, and examiner information indicating a name of an examiner in charge of the examination.

A ninth medical information management server according to the present invention is the eighth medical information management server that further includes a second determination section, wherein the provision request receiving section is capable of receiving physician identification information from the third terminal device in addition to the authentication key information, the patient identification information, and the information indicating a request for provision of examination result information. The second determination section, upon the first determination section determining match, performs determination as to whether or not the physician identification information corresponding to the examination result information stored in the storage region corresponding to the authentication key information matches the physician identification information received by the provision request receiving section. The information providing section transmits, upon the second determination section determining match, the examination result information containing the auxiliary information stored in the storage region corresponding to the received authentication key information to the third terminal device. When the provision request receiving section receives no physician identification information from the third terminal device or upon the second determination section determining non-match, the information providing section deletes the auxiliary information contained in the examination result information stored in the storage region corresponding to the received authentication key information and transmits the examination result information from which the auxiliary information has been deleted to the third terminal device.

A tenth medical information management sever according to the present invention is any of one of the fifth to ninth medical information management servers, wherein the physician identification information is information of a registration number of a physician registered in National Register of Physicians.

An eleventh medical information management server according to the present invention is any one of the fifth to tenth medical information management serves, wherein the patient identification information contains identification information used for identifying a medical institution and identification information used for identifying a patient in the medical institution.

A twelfth medical information management server according to the present invention is the eleventh medical information management server, wherein the identification information used for identifying a medical institution is a medical institution code.

A thirteenth medical information management server according to the present invention is any one of the first to twelfth medical information management servers, wherein the authentication key information corresponds to an authentication key that is a combination of any of an uppercase alphabet, a lowercase alphabet, and a numeral and of which the number of digits is a preset specific digit number or more.

A medical information management method according to the present invention is a medical information management method implemented by a medical information management server that is connected to a plurality of terminal devices through a network and that manages information on medical, care, and welfare services containing examination result information for medical use and includes receiving product number information, generating authentication key information, setting a storage region, and transmitting the authentication key information. In the receiving product number information, product number information of a medical product is received from a terminal device among the plurality of terminal devices through the network. In the generating authentication key information, authentication key information is generated based on the product number information when the product number information is received in the receiving product number information. In the setting a storage region, a storage region for storing examination result information is set in correspondence with the authentication key information generated in the generating authentication key information. In the transmitting the authentication key information, the authentication key information is transmitted to the terminal device through the network.

### [Advantageous Effects of Invention]

According to the medical information management server and the medical information management method in the present invention, leakage of the information on medical, care, and welfare services containing the examination result information can be prevented.

### [BRIEF DESCRIPTION OF DRAWINGS]

[FIG. 1] FIG. 1 is a diagram illustrating configuration of a medical information management system according to an embodiment of the present invention.
[FIG. 2] FIG. 2 is a block diagram illustrating functional configuration of the medical information management server illustrated in FIG. 1.
[FIG. 3] FIG. 3 is a diagram illustrating a product to which product number information that a product number receiving section of the medical information management server in FIG. 2 receives is attached.
[FIG. 4] FIG. 4 is a table indicating a content of examination result information that an examination result receiving section of the medical information management server in FIG. 2 receives.
[FIG. 5] FIG. 5 is a flowchart depicting a storage region setting process that the medical information management server in FIG. 2 performs.
[FIG. 6] FIG. 6 is a flowchart depicting an examination result recording process that the medical information management server in FIG. 2 performs.
[FIG. 7] FIG. 7 is a flowchart depicting an examination information providing process that the medical information management server in FIG. 2 performs.

### [DESCRIPTION OF EMBODIMENTS]

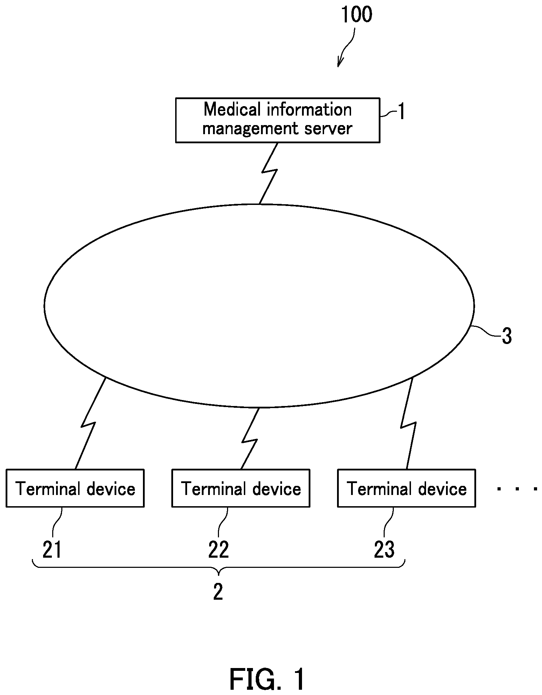

The following describes an embodiment of the present invention with reference to the drawings (FIGS. 1-7). Note that elements in the drawings that are the same or equivalent are labelled using the same reference signs and explanation thereof is not repeated.

First of all, a medical information management system 100 according to the present embodiment will be described with reference to FIG. 1. FIG. 1 is a diagram illustrating configuration of the medical information management system 100 according to the present embodiment. The medical information management system 100 includes a medical information management server 1 and a plurality of terminal devices 2.

The medical information management server 1 is connected to the plurality of terminal devices 2 in a communicable manner through a network 3 such as the Internet and manages information on medical, care, and welfare services containing examination result information for medical use. The medical information management server 1 includes a central processing unit (CPU), a read only memory (RAM), a random access memory (RAM), and a hard disk drive (HDD). The "information on medical, care, and welfare services" in the present embodiment includes medical service information, care service information, and welfare service information. Specifically, the term "information on medical, care, and welfare services" means information indicating for example service contents and results provided to a patient from for example a physician, a nurse, a pharmacist, a medical technician, or a care worker who are engaged in medical treatment, nursing, and/or care. Further specifically, the "information on medical, care, and welfare services" contains for example diagnosis result information that is information of a result of diagnosis by a physician, patient's condition information that is information of for example body temperature and blood pressure of a patient measured by a nurse, examination result information that is information of a result of an examination carried out by a medical technician, and care content information that is information of care provided by a care worker. The present embodiment describes a situation in which the "information on medical, care, and welfare services" is examination result information.

Terminal devices 21, 22, and 23 each are a personal computer including a barcode reader and reads product number information of a medical product using the barcode reader and transmits the read product number information to the medical information management server 1. The terminal devices 2 each further transmit examination result information for medical use to the medical information management server 1 so that the examination result information is stored in the HDD of the medical information management server 1. The terminal devices 2 each still further transmit information indicating a request for provision of examination result information to the medical information management server 1.

Note that the terminal devices 21, 22, and 23, which have substantially the same configuration, may be referred to collectively as terminal devices 2. Furthermore, the terminal device 21 corresponds to a "first terminal device". The terminal device 22 corresponds to a "second terminal device". The terminal device 23 corresponds to a "third terminal device".

Transmission and receipt of various information between the medical information management server 1 and the terminal devices 2 are performed via email in the present embodiment. In the above configuration, transmission and receipt of various information between the medical information management server 1 and the terminal devices 2 can be achieved through simple configuration. For example, email address information of the medical information management server 1 is contained in barcode information indicated by a barcode 43 attached to a medical product. The terminal devices 2 each acquire the email address information of the medical information management server 1 contained in the barcode information using the barcode reader not illustrated.

The present embodiment describes a configuration in which transmission and receipt of various information between the medical information management server 1 and the terminal devices 2 are performed via email. However, another method may be adopted for transmission and receipt of various information between the medical information management server 1 and the terminal devices 2. For example, the terminal devices 2 each may receive and transmit various information via a homepage of the medical information management server 1. In the above configuration, a homepage address of the medical information management server 1 is preferably contained in the barcode information attached to the medical product.

Furthermore, the present embodiment describes a configuration in which the terminal devices 2 each are a personal computer. However, any of the terminal devices 2 may be a tablet computer or a smartphone, for example.

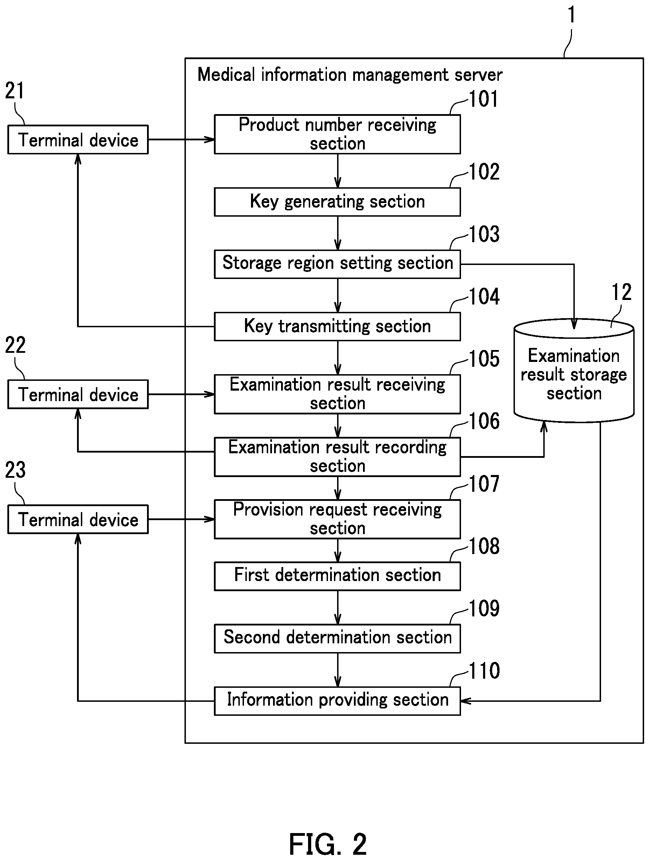

Functional configuration of the medical information management server 1 will be described next with reference to FIG. 2. FIG. 2 is a block diagram illustrating the functional configuration of the medical information management server 1 illustrated in FIG. 1. As illustrated in FIG. 2, the medical information management server 1 includes a product number receiving section 101, a key generating section 102, a storage region setting section 103, a key transmitting section 104, an examination result receiving section 105, an examination result recording section 106, a provision request receiving section 107, a first determination section 108, a second determination section 109, an information providing section 110, and an examination result storage section 12.

Note that the CPU of the medical information management server 1 reads out and executes control programs stored in the ROM (or the HDD) to function as the product number receiving section 101, the key generating section 102, the storage region setting section 103, the key transmitting section 104, the examination result receiving section 105, the examination result recording section 106, the provision request receiving section 107, the first determination section 108, the second determination section 109, and the information providing section 110. Furthermore, the CPU of the medical information management server 1 reads out and executes control programs stored in the ROM (or the HDD) to cause the HDD to function as the examination result storage section 12.

The storage region setting section 103 sets a storage region for storing examination result information for medical use in the examination result storage section 12 in correspondence with authentication key information generated by the key generating section 102. Further, the examination result storage section 12 stores for example examination result information received by the examination result receiving section 105 from a terminal device 2 (for example, the terminal device 22) in a storage region corresponding to authentication key information received by the examination result receiving section 105. Yet, the information providing section 110 reads out the examination result information stored in the storage region of the examination result storage section 12 corresponding to the authentication key information received by the provision request receiving section 107.

The first terminal device 21 acquires product number information of a medical product and the email address information of the medical information management server 1 that are contained in the barcode information using the barcode reader not illustrated. Further, the first terminal device 21 transmits the product number information addressed to the email address of the medical information management server 1.

The first terminal device 21 is a terminal device operated by for example a physician who administers medical treatment using the medical product.

The product number receiving section 101 receives the product number information of the medical product from the first terminal device 21 among the plurality of terminal devices 2 through the network 3. Specifically, the product number receiving section 101 receives the product number information in a manner to receive an email containing the product number information from the first terminal device 21.

When the product number receiving section 101 receives the product number information, the key generating section 102 generates authentication key information based on the product number information. The authentication key information is key information used for checking access authentication to a storage region set by the storage region setting section 103. The above storage region is a storage region included in the examination result storage section 12.

The storage region setting section 103 sets, in the examination result storage section 12, a storage region for storing examination result information in correspondence with the authentication key information generated by the key generating section 102.

The key transmitting section 104 transmits to the first terminal device 21 the authentication key information generate by the key generating section 102 through the network 3. Specifically, the key transmitting section 104 transmits the authentication key information in a manner to transmit an email containing the authentication key information to the first terminal device 21. For example, the key transmitting section 104 transmits an email containing the authentication key information to the first terminal device 21 in response to an email containing the product number information received by the product number receiving section 101. That is, the medical information management server 1 acquires mail address information of the first terminal device 21 through the product number receiving section 101 receiving the email containing the product number information from the first terminal device 21.

As described above, the authentication key information generated by the key generating section 102 is transmitted to the first terminal device 21 when the product number receiving section 101 receives the product number information. Generation of the authentication key information can result in prevention of leakage of the examination result information.

Furthermore, the authentication key information is transmitted to the first terminal device 21 via email, thereby reducing limitation on an authentication key corresponding to the authentication key information, such as the number of digits and usability of alphabet characters and numerals. For example, the number of digits of the authentication key can be set to be a preset specific number (for example, 20 digits) or more. Also, the authentication key can be a combination of any of a numeral, an uppercase alphabet and a lowercase alphabet. In the above configuration, even if someone would take a peep into a screen displaying the authentication key, it is rather difficult to memorize the authentication key, thereby preventing leakage of the authentication key.

The present embodiment describes a configuration in which the number of digits of the authentication key is a preset specific number (for example, 20 digits) or more and the authentication key is a combination of any of an uppercase alphabet, a lowercase alphabet, and a numeral. Leakage of the authentication key can be prevented in the above configuration.

The present embodiment describes but is not limited to a configuration in which the number of digits of the authentication key is the preset specific number or more (for example, 20 digits) and the authentication key is a combination of any of an uppercase alphabet, a lowercase alphabet, and a numeral. It is possible to impose either limitation of the number of digits of the authentication key being the preset specific number or more (for example, 20 digits) or the authentication key being a combination of any of an uppercase alphabet, a lowercase alphabet, and a numeral. The larger the number of digits of the authentication key is, the more hardly leakage of the authentication key occurs. The more the types of letters composing the authentication key is, the more hardly leakage of the authentication key occurs.

Furthermore, the first terminal device 21 receives the authentication key information by receiving an email from the medical information management server 1. In the above configuration, a user of the first terminal device 21 can acquire the authentication key information reliably in a simple manner.

The second terminal device 22 transmits the examination result information to the medical information management server 1 via email through the network 3. Note that the second terminal device 22 acquires the authentication key information and the email address information of the medical information management server 1 from the first terminal device 21. For example, the second terminal device 22 acquires the authentication key information and the email address information of the medical information management server 1 in a manner that the first terminal device 21 transmits to the second terminal device 22 an email containing the authentication key information received from the medical information management server 1.

Moreover, the second terminal device 22 is a terminal device operated by for example a physician who has instructed an examination corresponding to examination result information. The above examination result information contains, as auxiliary information, medical institution information indicating a medical institution in which the examination has been carried out, contact information indicating a contact address of the medical institution, and examiner information indicating a name of an examiner in charge of the examination.

The present embodiment describes a configuration in which the auxiliary information of the examination result information includes the medical institution information, the contact information, and the examiner information. However, it is only required in the present embodiment that the auxiliary information of the examination result information includes at least one of the medical institution information, the contact information, and the examiner information.

The examination result receiving section 105 receives the authentication key information, patient identification information used for identifying a patient subjected to the examination, physician identification information for identifying a physician who has instructed the examination, and the examination result information from the second terminal device 22 among the plurality of terminal devices 2 through the network 3. Specifically, the examination result receiving section 105 receives an email containing the authentication key information, the patient identification information, the physician identification information, and the examination result information from the second terminal device 22.

The patient identification information in the present embodiment is identification information of a combination of for example identification information used for identifying a medical institution such as a hospital and identification information used for identifying a patient in the medical institution. The physician identification information in the present embodiment is for example information of a registration number of a physician in the National Register of Physicians. Note that registration numbers of physicians in the National Register of Physicians are numbers assigned to the respective physicians in order by Ministry of Health, Labor and Welfare and recorded on a physician's license. The identification information used for identifying a medical institution is for example a "medical institution code".

As such, physician identification information herein is the physician identification information as described above. In the above configuration, appropriate setting of the physician identification information can be done easily. Furthermore, the patient identification information contains the identification information used for identifying a medical institution and the identification information used for identifying a patient in the medical institution. In the above configuration, appropriate setting of the patient identification information can be done easily.

When the examination result receiving section 105 receives the authentication key information, the patient identification information, the physician identification information, and the examination result information, the examination result recording section 106 stores the examination result information in correspondence with the patient identification information and the physician identification information in a storage region of the examination result storage section 12 corresponding to the authentication key information. However, in a situation in which the storage region corresponding to the authentication key information received by the examination result receiving section 105 is not set in the examination result storage section 12, the examination result recording section 106 transmits to the second terminal device 22 a message indicating that the authentication key information received by the examination result receiving section 105 is wrong.

As described above, when the examination result receiving section 105 receives the authentication key information, the patient identification information, the physician identification information, and the examination result information, the examination result recording section 106 stores the examination result information in correspondence with the patient identification information and the physician identification information in the storage region of the examination result storage section 12 corresponding to the authentication key information. Meanwhile, the authentication key information is generated based on the product number information of the medical product. In the above configuration, examination result information of a patient subjected to an examination using the medical product corresponding to the product number information can be stored in the examination result storage section 12.

In other words, through purchase of a medical product and an examination using the purchased medical product, examination result information of a patient subjected to the examination can be stored in the examination result storage section 12. This can increase convenience to medical personnel and the patient.

Furthermore, in a situation in which no storage region corresponding to the authentication key information received by the examination result receiving section 105 is set in the examination result storage section 12, the examination result recording section 106 transmits to the second terminal device 22 a message indicating that the authentication key information received by the examination result receiving section 105 is wrong. In the above configuration, a user of the second terminal device 22 can recognize that the above authentication key information is wrong. As a result, convenience to the user can be increased.

The third terminal device 23 transmits the authentication key information, the patient identification information, and information indicating a request for provision of examination result information to the medical information management server 1 through the network 3. Specifically, the third terminal device 23 transmits an email containing the authentication key information, the patient identification information, and the information indicating a request for provision of examination result information to the medical information management server 1. For example, the third terminal device 23 acquires the authentication key information and the email address information of the medical information management server 1 through the first terminal device 21 transferring an email containing the authentication key information received from the medical information management server 1 to the third terminal device 23.

Moreover, the third terminal device 23 is a terminal device operated by for example a person who requests provision of examination result information. The person who requests provision of examination result information is for example a patient oneself corresponding to the patient identification information, a physician who has instructed an examination corresponding to the examination result information, or a physician different from the physician who has instructed an examination corresponding to the examination result information.

Note that it is necessary for a person other than the physician who has instructed an examination corresponding to the examination result information (for example, a patient oneself corresponding to the patient identification information or a physician different from the physician who has instructed an examination corresponding to the examination result information) to acquire the authentication key information. The patient oneself corresponding to the patient identification information receives the authentication key information from the physician who has instructed an examination corresponding to the examination result information for example at the end of the examination or in examination result explanation or medical consultation. The physician different from the physician who has instructed an examination corresponding to the examination result information receives the authentication key information for example from the patient oneself corresponding to the patient identification information or the physician who has instructed an examination corresponding to the examination result information.

The provision request receiving section 107 receives the authentication key information, the patient identification information, and the information indicating a request for provision of examination result information from the third terminal device 23 through the network 3. The provision request receiving section 107 can further receive the physician identification information from the third terminal device 23, in addition to the authentication key information, the patient identification information, and the information indicating a request for provision of examination result information. Specifically, the provision request receiving section 107 receives from the third terminal device 23 an email containing the authentication key information, the patient identification information, the physician identification information, and the information indicating a request for provision of examination result information.

When the provision request receiving section 107 receives the information indicating a request for provision of examination result information, the first determination section 108 determines whether or not the received patient identification information matches patient identification information associated with the received authentication key information. The patient identification information associated with the authentication key information specifically is patient identification information corresponding to the examination result information stored in the storage region corresponding to the authentication key information. The patient identification information corresponding to the examination result information is patient identification information corresponding to a patient who is a subject of the examination indicated in the examination result information. Note that the storage region is a storage region in the examination result storage section 12.

Upon the first determination section 108 determining match, the second determination section 109 determines whether or not the physician identification information corresponding to the examination result information matches the physician identification information received by the provision request receiving section 107. Note that the above examination result information is the examination result information stored in the storage region corresponding to the authentication key information.

Upon the first determination section 108 determining match, the information providing section 110 transmits the examination result information to the third terminal device 23. Note that the above examination result information is the examination result information stored in the storage region corresponding to the authentication key information received by the provision request receiving section 107. Moreover, upon the second determination section 109 determining match, the information providing section 110 transmits the examination result information containing the auxiliary information to the third terminal device 23. By contrast, in a situation in which the provision request receiving section 107 receives no physician identification information or upon the second determination section 109 determining non-match, the provision request receiving section 107 deletes the auxiliary information contained in the examination result information and transmits the examination result information from which the auxiliary information has been deleted to the third terminal device 23.

Specifically, upon the first determination section 108 determining match, the information providing section 110 transmits an email containing the examination result information to the third terminal device 23. Note that the above examination result information is the examination result information stored in the storage region corresponding to the authentication key information received by the provision request receiving section 107. Moreover, upon the second determination section 109 determining match, the information providing section 110 transmits an email containing the examination result information containing the auxiliary information to the third terminal device 23. By contrast, in a situation in which the provision request receiving section 107 receives no physician identification information or upon the second determination section 109 determining non-match, the information providing section 110 transmits to the third terminal device 23 an email containing the examination result information from which the auxiliary information has been deleted.

As described above, the examination result information stored in the storage region corresponding to the authentication key information received by the provision request receiving section 107 is transmitted to the third terminal device 23 upon the first determination section 108 determining match. In the above configuration, leakage of the examination result information can be prevented. Note that the above examination result information is the examination result information stored in the storage region corresponding to the authentication key information received by the provision request receiving section 107. In other words, the authentication key information and the patient identification information that correspond to the examination result information are essential to acquisition of the examination result information. As such, leakage of the examination result information can be prevented.

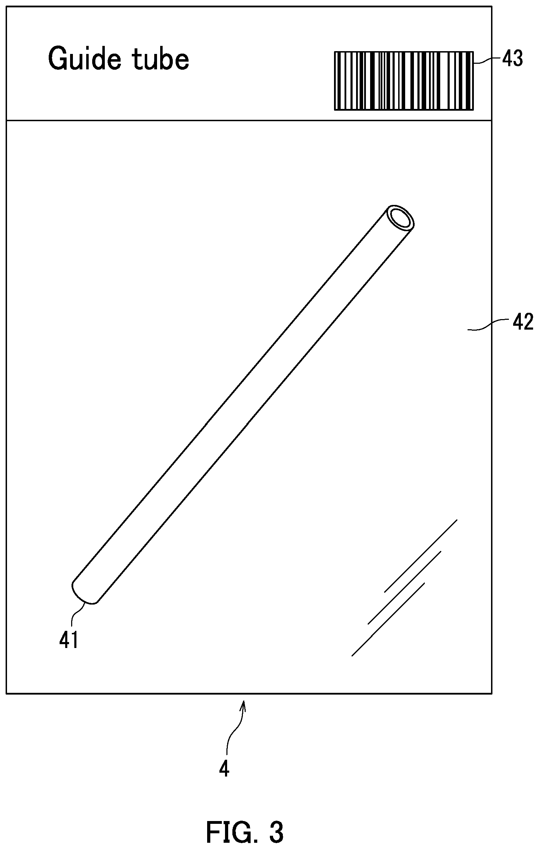

The email address information of the medical information management server 1 and the product number information will be described next with reference to FIG. 3. FIG. 3 is a diagram illustrating a product 4. Product number information that the product number receiving section 101 of the medical information management server 1 in FIG. 2 receives is assigned to the product 4. The product 4 corresponds to an example of the "medical product".

The product 4 includes a product body (guide tube) 41 and a package 42. The guide tube 41 is made from for example silicone, polyethylene, or vinyl chloride and is a tube to be inserted into a nostril to guide a nasal endoscope to a throat (or a larynx).

The package 42 is a package made from for example polyethylene, polypropylene, or vinyl chloride in which the guide tube 41 is packaged. The package 42 has an upper part to which a barcode 43 is attached. The barcode 43 indicates barcode information including product number information of the guide tube 41 and the email address information of the medical information management server 1.

The first terminal device 21 acquires through the non-illustrated barcode reader the email address information of the medical information management server 1 and the product number information of the guide tube 41 that are contained in the barcode information indicated on the barcode 43. The first terminal device 21 then transmits an email containing the product number information of the guide tube 41 contained in the barcode information indicated on the barcode 43 to the medical information management server 1 (an email address contained in the barcode information indicated on the barcode 43) through the network 3.

The present embodiment describes but is not limited to a configuration in which the barcode 43 is attached to the package 42 for the product body 41. It is only required that the product body 41 to which a code is attached. For example, a piece of paper on which a two-dimensional barcode (or barcode) is printed may be enclosed in the package 42. The two-dimensional barcode is for example a QR code (registered Japanese trademark). The piece of paper on which the two-dimensional barcode is printed is preferably folded so that the two-dimensional barcode is non-readable from the outside. In the above configuration, a user is enabled to read the two-dimensional barcode only after the package 42 is opened. This can prevent leakage of information contained in the two-dimensional barcode (the product number information and the email address information).

Furthermore, the product 4 is used for endoscopy. Furthermore, the product when used in an endoscopic examination for a single patient is not used in the endoscopic examination for another patient. In other words, the product 4 is disposed of after an endoscopic examination. In the above case, the information (the product number information and the email address address) contained in the barcode 43 attached to the package 42 for the product body 41 is made correspondence with a single patient subjected to an endoscopic examination using the product body 41. In other words, the product number information contained in the barcode 43 is made correspondence with a single piece of patient identification information. In the above configuration, the examination result recording section 106 described with reference to FIG. 2 can record an examination result of an endoscopic examination in a storage region set in correspondence with the authentication key information associated with the product number information contained in the barcode 43. Thus, the product number information contained in the barcode 43 can be used for setting a region where the examination result of the patient is to be stored. Note that the product body 41 corresponding to the barcode 43 is used in the examination for the patient.

The present embodiment describes but is not limited to a configuration in which the product 4 is disposed of once used in an examination. The product 4 once used in an examination for a patient may be used in a repetitive manner only for the same patient.

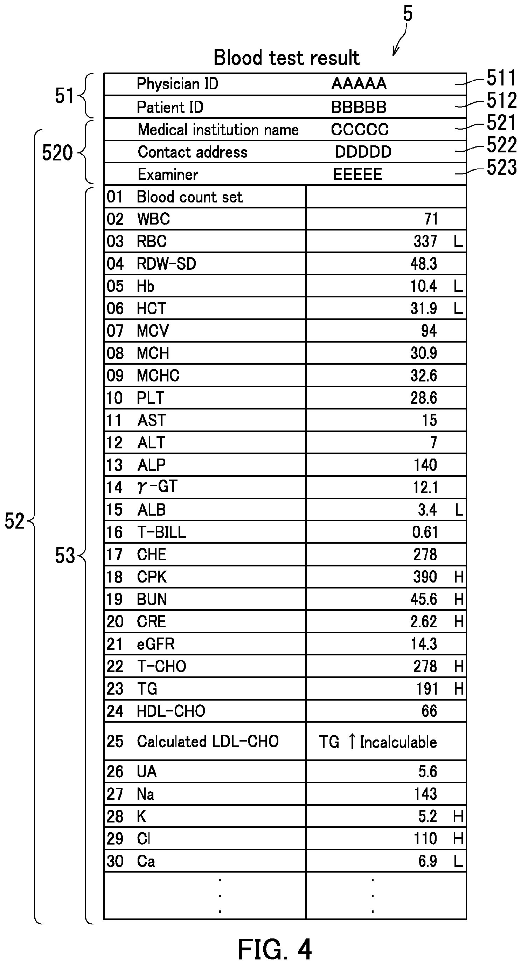

The examination result information 5 will be described next with reference to FIG. 4. FIG. 4 indicates a configuration of the examination result information 5. The examination result information 5 is received by the examination result receiving section 105 of the medical information management server 1 illustrated in FIG. 2. Further, FIG. 4 is a table listing an example of blood examination result information that is the examination result information 5. The examination result information 5 contains essential information 51 and examination specific information 52. The examination specific information 52 contains auxiliary information 520 and examination item information 53.

The essential information 51 is information necessarily contained in the examination result information 5 and includes physician identification information 511 and patient identification information 512. The auxiliary information 520 includes medical institution name information 521, contact address information 522, and examiner information 523. The examination item information 53 is examination result information on an examination item by an item by item basis.

As described above, the examination result information 5 contains the auxiliary information 520 including for example the medical institution name information 521, the contact address information 522, and the examiner information 523. In the above configuration, a physician can inquire of for example an examiner about the content of the examination item information 53 as necessary, thereby enhancing usability for the physician.

In a situation in which the examination result information 5 indicated in FIG. 4 is stored in the examination result storage section 12, the first and second determination sections 108 and 109 perform the following determination. First, when the provision request receiving section 107 receives information indicating a request for provision of examination result information, the first determination section 108 determines whether or not the patient identification information received by the provision request receiving section 107 matches the patient identification information 512 corresponding to the above examination result information. Note that the above examination result information is examination result information stored in a storage region corresponding to the received authentication key information. Upon the first determination section 108 determining match, the second determination section 109 determines whether or not the physician identification information received by the provision request receiving section 107 matches the physician identification information 511 corresponding to the above examination result information. Note that the above examination result information is the examination result information stored in the storage region corresponding to the authentication key information.

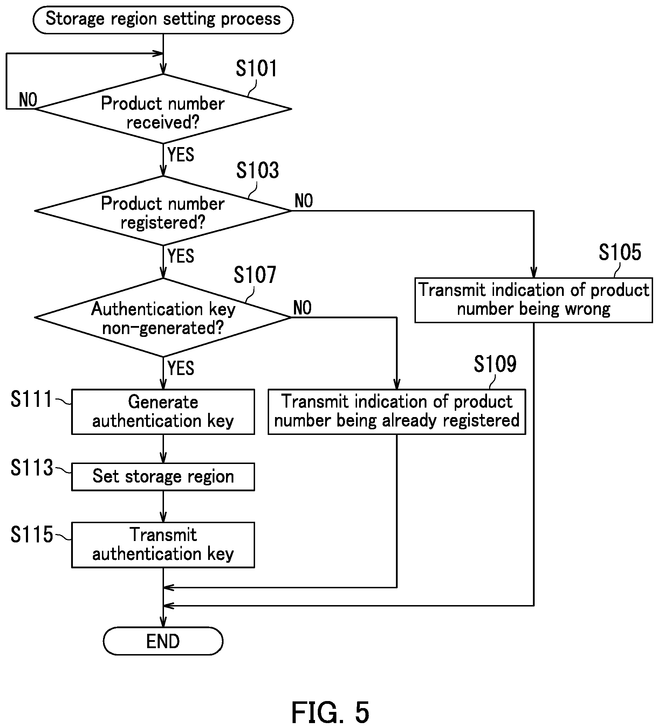

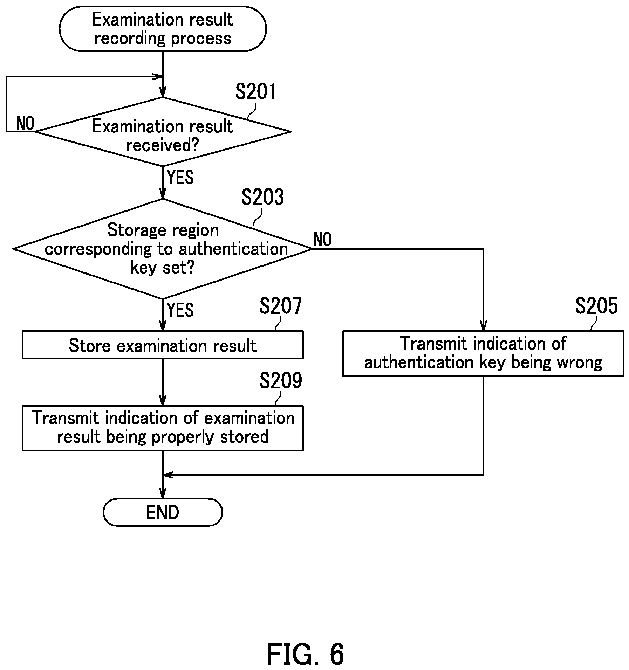

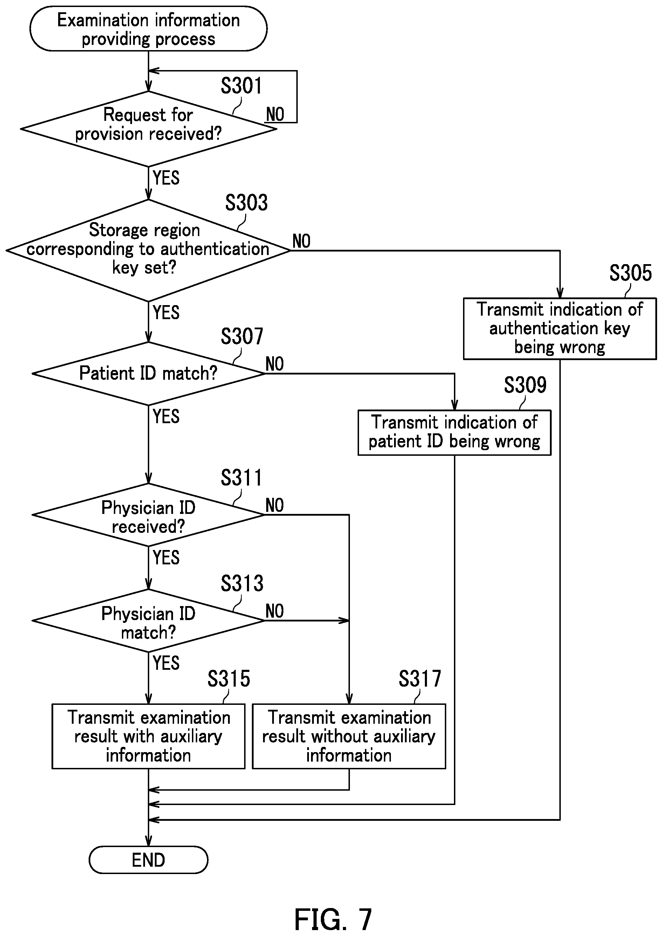

Operation of the medical information management server 1 will be described next with reference to FIGS. 5-7. FIG. 5 is a flowchart depicting a storage region setting process that the medical information management server 1 in FIG. 2 performs. FIG. 6 is a flowchart depicting an examination result recording process that the medical information management server 1 in FIG. 2 performs. FIG. 7 is a flowchart depicting an examination information providing process that the medical information management server in FIG. 2 performs.

The "storage region setting process" will be described first with reference to FIG. 5. The "storage region setting process" is a process of generating an authentication key upon receipt of product number information from the first terminal device 21 and setting a storage region in the examination result storage section 12 in correspondence with the generated authentication key. FIG. 7 is a flowchart depicting an examination information providing process that the medical information management server in FIG. 2 performs.

First, the product number receiving section 101 performs determination as to whether or not product number information is received from the first terminal device 21 (Step S101). Upon determination that no product number information is received (NO at Step S101), processing is held on standby. Upon determination that product number information is received (YES at Step S101), the key generating section 102 performs determination as to whether or not the product number information received at Step S101 matches any piece of pre-registered product number information, that is, whether or not it is registered (Step S103).

Upon determination that the product number information receive at Step S101 is not registered (NO at Step S103), the key generating section 102 transmits information indicating that the product number information received at Step S101 is wrong to the first terminal device 21 (Step S105). The process then ends. Upon the key generating section 102 determining that it is registered (YES at Step S103), the key generating section 102 performs determination as to whether or not an authentication key corresponding to the product number information received at Step S101 is non-generated (Step S107).

Upon determination that the authentication key corresponding to the product number information received at Step S101 is already generated (NO at S107), information indicating that the product number information received at Step S101 has been already used is transmitted (Step S109). The process then ends. Note that the wording "the product number information has been already used" means that an authentication key corresponding to the product number information has been already generated and a corresponding storage region has been already set.

Upon determination that an authentication key corresponding to the product number information received at Step S101 has not been generated (YES at Step S107), the key generating section 102 generates an authentication key corresponding to the product number information received at Step S101 (step S111). Then, the storage region setting section 103 sets a storage region for storing examination result information is set in the examination result storage section 12 in correspondence with the authentication key generated at Step S111 (Step S113). Next, the key transmitting section 104 transmits the authentication key information generated at Step S111 to the first terminal device 21 (step S115). The processing then ends.

As described above, when it is determined at Step S107 that an authentication key corresponding to the product number information has been already generated, the information indicating that the product number information has been already used is transmitted and the process ends. In the above configuration, single use of the guide tube 41 can be ensured. Note that Step S101 corresponds to "receiving product number information" and Step S111 corresponds to "generating authentication key information". Furthermore, Step S113 corresponds to "setting a storage region" and Step S115 corresponds to "transmitting the authentication key information".

The "examination result recording process" will be described next with reference to FIG. 6. The "examination result recording process" is a process of receiving examination result information from the second terminal device 22 and storing the received examination result information in the storage region set by the examination result storage section 12 at Step S113 in FIG. 5. Note that the examination result information in the following description is the examination result information 5 indicated in FIG. 4.

First, the examination result receiving section 105 performs determination as to whether or not authentication key information, patient identification information 512, physician identification information 511, and examination specific information 52 are received from the second terminal device 22 (Step S201). Upon determination at Step S201 that they are not received (NO at Step S201), the processing is held on standby. Upon determination at Step S201 that they are received (YES at Step S201), the examination result recording section 106 performs determination as to whether or not a storage region corresponding to the authentication key information received at Step S201 is set in the examination result storage section 12 (Step S203).

Upon determination that the storage region is not set in the examination result storage section 12 (NO at Step S203), the examination result recording section 106 transmits information indicating that the authentication key information received at Step S201 is wrong to the second terminal device 22 (Step S205). The process then ends. Upon determination that the storage region is set in the examination result storage section 12 (YES at Step S203), the examination result recording section 106 stores the examination specific information 52 containing the auxiliary information 520 in correspondence with the patient identification information 512 and the physician identification information 511 in the storage region corresponding to the authentication key information received at Step S201 (Step S207). The examination result recording section 106 then transmits information indicating that the transmitted examination specific information 52 is properly stored in the storage region of the examination result storage section 12 to the second terminal device 22 (Step S209). The process then ends.

The "examination information providing process" will be described next with reference to FIG. 7. The "examination information providing process" means a process of transmitting the examination specific information 52 stored in the examination result storage section 12 at Step S207 in FIG. 6 to the third terminal device 23 when information indicating a request for provision of examination specific information 52 is received from the third terminal device 23.

First, the provision request receiving section 107 performs determination as to whether or not authentication key information, patient identification information, and information indicating a request for provision of examination result information are received from the third terminal device 23 (Step S301). Upon determination that the authentication key information, the patient identification information, and the information indicating a request for provision of examination result information are not received (NO at Step S301), the processing is held on standby. Upon determination that the authentication key information, the patient identification information, and the information indicating a request for provision of examination result information are received (YES at Step S301), the first determination section 108 performs determination as to whether or not a storage region corresponding to the authentication key information received at Step S301 is set in the examination result storage section 12 (Step S303).

Upon determination that the storage region is not set in the examination result storage section 12 (NO at Step S303), the first determination section 108 transmits information indicating that the authentication key information received at Step S301 is wrong to the third terminal device 23 (Step S305). The process then ends. Upon determination that the storage region is set in the examination result storage section 12 (YES at Step S303), the first determination section 108 performs determination as to whether or not the patient identification information received at Step S301 matches the patient identification information 512 associated with the authentication key information received at Step S301 (Step S307).

Upon determination at Step S307 that it does not match the patient identification information 512 (NO at Step S307), the first determination section 108 transmits information indicating that the received patient identification information is wrong to the third terminal device 23 (Step S309). The process then ends. Upon determination at Step S307 that it matches the patient identification information 512 (YES at Step S307), the second determination section 109 performs determination as to whether or not physician identification information is received at Step S301 (Step S311). Upon determination that no physician identification information is received (NO at Step S311), the process proceeds to Step S317. Upon determination that physician identification information is received (YES at Step S311), the second determination section 109 performs determination as to whether or not the physician identification information 511 corresponding to the examination specific information 52 matches the physician identification information received at Step S301 (Step S313). Note that the examination specific information 52 is examination specific information 52 stored in a storage region corresponding to the authentication key information received at Step S301.

Upon determination that it does not match the physician identification information received at Step S301 (NO at Step S313) or upon determination at Step S311 resulting in NO, the information providing section 110 deletes the auxiliary information 520 contained in the examination specific information 52 and transmits the examination specific information 52 from which the auxiliary information 520 has been deleted to the third terminal device 23 (Step S317). The process then ends. Upon determination that it matches the physician identification information received at Step S301 (YES at S313), the examination specific information 52 containing the auxiliary information 520 is transmitted to the third terminal device 23 (Step S315). The process then ends.

As described above, when the physician identification information 511 corresponding to the examination result information 5 does not match the received physician identification information, the auxiliary information 520 contained in the examination result information is deleted and the examination result information from which the auxiliary information 520 has been deleted is transmitted to the third terminal device 23. In the above configuration, leakage of the auxiliary information 520 contained in the examination result information 5 can be prevented. Note that the examination result information 5 is examination result information 5 stored in a storage region corresponding to the received authentication key information.

Specifically, for example, a physician who has instructed an examination through which examination result information is generated can recognize auxiliary information 520 contained in examination specific information 52 through transmission of physician identification information of physician's own to the medical information management server 1. The auxiliary information 520 includes the medical institution name information 521, the contact address information 522, and the examiner information 523. In the above configuration, an inquiry can be made to a corresponding examiner in a situation in which some question relating to the examination item information 53 arises.

By contrast, for example, a patient oneself (or a pharmacist or another physician) who does not know the physician identification information 511 is disabled to recognize the auxiliary information 520 contained in the examination specific information 52. In the above configuration, leakage of the medical institution name information 521, the contact address information 522, and the examiner information 523 included in the auxiliary information 520 can be prevented.

An embodiment of the present invention has been described so far with reference to the drawings. However, the present disclosure is of course not limited to the above embodiment and may be practiced in various forms without deviating from the essence thereof (for example, as explained below in sections (1) and (4)). The drawings schematically illustrate elements of configuration in order to facilitate understanding and properties of elements of configuration illustrated in the drawings, such as thickness, length, and number thereof, may differ from actual properties thereof in order to facilitate preparation of the drawings. Shapes, dimensions, etc. of the elements of configuration given in the above embodiment are merely examples that do not impart any particular limitations and may be altered in various ways, so long as such alterations do not substantially deviate from the configuration of the present disclosure.
(1) The present embodiment describes a configuration with a single piece of the examination result information 5 for the sake of convenience. However, plural pieces of examination result information may be stored in the examination result storage section 12. In the above configuration, the second determination section 109 performs the determination for each piece of the examination result information.
(2) The present embodiment describes a configuration in which the examination result information 5 is blood examination result information. However, it is only required that the examination result information is examination result information for medical use. For example, the examination result information may be urinalysis result information, computed tomography (CT) result information, magnetic resonance imaging (MRI) result information, or medical checkup result information including for example height and weight.
(3) The present embodiment describes a configuration in which the network 3 is the Internet. However, the network 3 may be another network. For example, the network 3 may be a local area network (LAN) or a wide area network (WAN).
(4) The present embodiment describes a configuration in which the key generating section 102 generates authentication key information based on product number information once the product number receiving section 101 receives the product number information. However, generation of authentication key information based on the product number information by the key generating section 102 may be triggered by another condition. For example, the key generating section 102 may generate authentication key information based on the product number information when a contract for use of the medical information management server 1 is established between a medical personnel and an administrator who administers the medical information management server 1. Alternatively, for example, the key generating section 102 may generate authentication key information based on the product number information when a physician transmits examination result information to the medical information management server 1.

### [INDUSTRIAL APPLICABILITY]

The present invention is applicable to a medical information management server and a medical information management method for managing examination result information for medical use.

### [REFERENCE SINGS LIST]

- 100: medical information management system
- 1: medical information management server
- 101: product number receiving section
- 102: key generating section
- 103: storage region setting section
- 104: key transmitting section
- 105: examination result receiving section
- 106: examination result recording section
- 107: provision request receiving section
- 108: first determination section
- 109: second determination section
- 110: information providing section
- 12: examination result storing section
- 2: terminal device
- 3: network
- 4: product (medical product)
- 41: guide tube
- 42: package
- 43: barcode
- 5: examination result information
- 51: essential information
- 511: physician identification information
- 512: patient identification information
- 52: examination specific information
- 520: auxiliary information
- 521: medical institution name information
- 522: contact address information
- 523: examiner information
- 53: examination item information

## Claims

1. A medical information management server that is connected to a plurality of terminal devices in a communicable manner through a network and that manages information on medical, care, and welfare services containing examination result information for medical use, the medical information management server comprising:
a product number receiving section configured to receive product number information of a medical product from a first terminal among the plurality of terminal devices through the network;
a key generating section configured to generate authentication key information based on the product number information when the product number receiving section receives the product number information;
a storage region setting section configured to set a storage region for storing examination result information in correspondence with the authentication key information generated by the key generating section; and
a key transmitting section configured to transmit the authentication key information to the first terminal through the network.

2. The medical information management server according to claim 1, wherein
the medical product to which a code is attached,
the code indicates information containing the product number information and email address information of the medical information management server, and
the product number receiving section receives the product number information in a manner to receive an email containing the product number information from the first terminal device.

3. The medical information management server according to claim 2, wherein
the code is a barcode attached to a package of the medical product.

4. The medical information management server according to claim 2 or 3, wherein
the medical product is used for an examination, and
once the medical product is used in the examination for a single patient, the medical product is not used in the examination for another patient.

5. The medical information management server according to any one of claims 1-4, further comprising:
an examination result receiving section configured to receive authentication key information, patient identification information, physician identification information, and examination result information from a second terminal device among the plurality of terminal devices through the network, the patient identification information being information used for identifying a patient subjected to an examination, the physician identification information being information used for identifying a physician who has instructed the examination; and
an examination result recording section configured to, when the examination result receiving section receives the authentication key information, the patient identification information, the physician identification information, and the examination result information, store the receive examination result information in a storage region corresponding to the received authentication key information in association with the received authentication key information, the received patient identification information, and the received physician identification information.

6. The medical information management server according to claim 5, wherein
in a situation in which when the examination result receiving section receives authentication key information, patient identification information, physician identification information, and examination result information, no storage region corresponding to the received authentication key information is set, the examination result recording section transmits a message indicating that the received authentication key information is wrong to the second terminal device.

7. The medical information management server according to claim 5 or 6, further comprising:
a provision request receiving section configured to receive authentication key information, patient identification information, and information indicating a request for provision of examination result information from a third terminal device among the plurality of terminal devices through the network;
a first determination section configured to, when the provision request receiving section receives the authentication key information, the patient identification information, and the information indicating a request for provision of examination result information, performs determination as to whether or not the received patient identification information matches the patient identification information associated with the received authentication key information; and
an information providing section configured to transmit examination result information stored in a storage region corresponding to the authentication key information received by the provision request receiving section to the third terminal device upon the first determination section determining match.

8. The medical information management server according to claim 7, wherein
the examination result information contains auxiliary information including at least one piece of medical institution information indicating a medical institution in which an examination has been carried out, contact address information indicating a contact address of the medical institution, and examiner information indicating a name of an examiner in charge of the examination.

9. The medical information management server according to claim 8, wherein
the provision request receiving section is capable of receiving physician identification information from the third terminal device in addition to the authentication key information, the patient identification information, and the information indicating a request for provision of examination result information,
the medical information management server further comprises a second determination section configured to, upon the first determination section determining match, perform determination as to whether or not the physician identification information corresponding to the examination result information stored in the storage region corresponding to the generated authentication key information matches the physician identification information received by the provision request receiving section, and
the information providing section
transmits, upon the second determination section determining match, the examination result information containing the auxiliary information stored in the storage region corresponding to the received authentication key information to the third terminal device, and
deletes, when the provision request receiving section receives no physician identification information from the third terminal device or upon the second determination section determining non-match, the auxiliary information contained in the examination result information stored in the storage region corresponding to the received authentication key information and transmits the examination result information from which the auxiliary information has been deleted to the third terminal device.

10. The medical information management server according to any one of claims 5-9, wherein
the physician identification information is information of a registration number of a physician registered in National Register of Physicians.

11. The medical information management server according to any one of claims 5-10, wherein
the patient identification information contains identification information used for identifying a medical institution and identification information used for identifying a patient in the medical institution.

12. The medical information management server according to claim 11, wherein
the identification information used for identifying a medical institution is a medical institution code.

13. The medical information management server according to any one of claims 1-12, wherein
the authentication key information corresponds to an authentication key that is a combination of any of an uppercase alphabet, a lowercase alphabet, and a numeral and of which the number of digits is a preset specific digit number or more.

14. A medical information management method implemented by a medical information management server that is connected to a plurality of terminal devices through a network and that manages information on medical, care, and welfare services containing examination result information for medical use, the medical information management method comprising:
receiving product number information of a medical product from a terminal among the plurality of terminal devices through the network;
generating authentication key information based on the product number information when the product number information is received in the receiving product number information;
setting a storage region for storing examination result information in correspondence with the authentication key information generated in the generating authentication key information; and
transmitting the authentication key information to the terminal through the network.
